# EUROPEAN PATENT SPECIFICATION

(11) **EP 1 254 216 B1**
(45) Date of publication and mention of the grant of the patent: **07.06.2006**
(21) Application number: 01907078.8
(22) Date of filing: 07.02.2001
(51) Int. Cl.: C12N 15/00

(54) **IMPROVED GALACTOSYLATION OF RECOMBINANT GLYCOPROTEINS**
VERBESSERTE GALACTOSYLIERUNG VON REKOMBINANTEN PROTEINEN
AMELIORATION DE LA GALACTOSYLATION DE GLYCOPROTEINES DE RECOMBINAISON

(30) Priority: 08.02.2000 US 181108 P; 23.03.2000 US 191641 P
(43) Date of publication of application: 06.11.2002
(73) Proprietor: GENENTECH, INC., South San Francisco California 94080 (US)
(72) Inventor: RYLL, Thomas, San Mateo, CA 94402 (US); WANG, Frank S., Morrisville, Noth Carolina 27560 (US)
(74) Representative: Kiddle, Simon John
(86) International application number: PCT/US2001/003945
(87) International publication number: WO 2001/059089

(56) References cited:
- WO-A-01/14569
- KEMPE T D ET AL: "STABLE MUTANTS OF MAMMALIAN CELLS THAT OVER PRODUCE THE 1ST 3 ENZYMES OF PYRIMIDINE NUCLEOTIDE BIOSYNTHESIS" CELL, vol. 9, no. (4 PART 1, 1976, pages 541-550, XP001012273 ISSN: 0092-8674 cited in the application
- LAVAL M ET AL: "OVERPRODUCTION OF THE FIRST THREE ENZYMES OF PYRIMIDINE NUCLEOTIDE BIOSYNTHESIS IN DROSOPHILA CELLS RESISTANT TO N PHOSPHONACETYL-L-ASPARTATE" EXPERIMENTAL CELL RESEARCH, vol. 163, no. 2, 1986, pages 381-395, XP001012272 ISSN: 0014-4827
- RYLL T ET AL: "IMPROVED ION-PAIR HIGH-PERFORMANCE LIQUID CHROMATOGRAPHIC METHOD FOR THE QUANTIFICATION OF A WIDE VARIETY OF NUCLEOTIDES AND SUGAR-NUCLEOTIDES IN ANIMAL CELLS" JOURNAL OF CHROMATOGRAPHY. BIOMEDICAL APPLICATIONS,ELSEVIER, AMSTERDAM,NL, vol. 570, no. 1, 18 September 1991 (1991-09-18), pages 77-88, XP001005650 ISSN: 0378-4347 cited in the application
- CARREY E A: "Key enzymes in the biosynthesis of purines and pyrimidines: Their regulation by allosteric effectors and by phosphorylation." BIOCHEMICAL SOCIETY TRANSACTIONS, vol. 23, no. 4, 1995, pages 899-902, XP001014381 655th Meeting of the Biochemical Society;Manchester, England, UK; July 18-21, 1995 ISSN: 0300-5127 cited in the application
- CARREY E A ET AL: "PHOSPHORYLATION AND ACTIVATION OF HAMSTER CARBAMYL PHOSPHATE SYNTHETASE II BY CYCLIC AMP-DEPENDENT PROTEIN KINASE A NOVEL MECHANISM FOR REGULATION OF PYRIMIDINE NUCLEOTIDE BIOSYNTHESIS" EMBO (EUROPEAN MOLECULAR BIOLOGY ORGANIZATION) JOURNAL, vol. 4, no. 13B, 1985, pages 3735-3742, XP001014379 ISSN: 0261-4189
- PARK H -S ET AL: "Expression of carbamoyl phosphate synthetase I and ornithine transcarbamoylase genes in Chinese hamster ovary dhfr-cells decreases accumulation of ammonium ion in culture media" JOURNAL OF BIOTECHNOLOGY,ELSEVIER SCIENCE PUBLISHERS, AMSTERDAM,NL, vol. 81, no. 2-3, 25 August 2000 (2000-08-25), pages 129-140, XP004210484 ISSN: 0168-1656

## Description

This application is being filed as a PCT application by GENENTECH, INC., a United States national and resident, designating all countries.

### RELATED APPLICATIONS

This application claims priority of United States provisional application Serial Number 60/181,108, filed February 8, 2000 and United States provisional application Serial Number 60/191,641, filed March 23, 2000.

### FIELD OF THE INVENTION

This invention relates to glycosylation of recombinant glycoproteins and more particularly the invention relates to a process of producing glycoproteins within cells having enhanced CAD activity.

### BACKGROUND OF THE INVENTION

Recombinant glycoproteins expressed by foreign genes in host cells are of major interest to the scientific community because of their potential value as prophylactics and therapeutics. Recombinant glycoproteins are composed of amino acid chains that display a series of complex and diverse oligosaccharide structures. While the amino acid sequence of recombinant glycoproteins can be reproduced consistently, the oligosaccharides side chains are often heterogeneous in both composition and structure. Rademacker et al., (1988) Annu. Rev. Biochem. 57:785-838; Spellman et al., (1989) J. Biol. Chem. 264(24):14100-14111; Spellman et al., (1991) Biochemistry 30:2395-2406; Kagawa et al., (1988) J. Biol. Chem. 263(33):17508-17515.

Heterogeneous oligosaccharide structure can have unfavorable affects on a glycoprotein's properties, including: glycoprotein function, conformation, solubility, specific activity, antigenicity and immunogenicity. Wittwer et al., (1990) Biochem. 29:4175-4180; Hart (1992) Curr. Op. Cell Biol., 4:1017-1023; Parekh (1991) Curr. Op. Struct. Biol., 1:750-754. Therefore, attention within the scientific community is focused on producing recombinant glycoproteins having both the correct amino acid sequence and that display a less heterogeneous oligosaccharide profile, preferably one that corresponds to the native protein. This is especially important in the production of therapeutics, where the Food and Drug Administration requires that the same oligosaccharide pattern be displayed on any biological therapeutic.

Several strategies have been employed to affect the composition of oligosaccharides in recombinant glycoproteins, these include: (1) production of recombinant host cell lines that over-express particular glycosyltransferases with a goal of enhanced biosynthesis; (2) preventing host cell oligosaccharide degradation by regulating glycohydrolytic enzymes; and (3) culturing recombinant glycoprotein producing host cells in the presence of free sugars and other substrates required for oligosaccharide production. However, there are drawbacks associated with these strategies. For example, glycosyltransferases may only be as efficient as the amount of oligosaccharide substrate within the cell; regulating oligosaccharide degradation may not be effective where the proteins are improperly glycosylated; and various proposed feeding strategies can be cost prohibitive and lead to detrimental feedback inhibition of relevant glycosyltransferases. There remains a need in the recombinant glycoprotein art for a method of inexpensively producing recombinant glycoproteins displaying consistent oligosaccharide profiles. Against this backdrop the present invention has been developed.

### SUMMARY OF THE INVENTION

The present invention provides improved galactosylation and sialyation of recombinant glycoproteins by enhancing *de novo* pyrimidine biosynthesis via enhanced Carbamoyl-phosphate synthase II (CPS II)/aspartate transcarbamoylase (ATCase)/dihydro-orotase (CAD) activity. It is a feature of the present invention that glycoproteins, produced in cells having enhanced CAD activity, display enhanced galactosylation. The invention is useful for the expression of glycoproteins from endogenous genes or from nucleic acids introduced into the cells under appropriate conditions. The present invention is especially useful, therefore, for recombinant expression of proteins having galactose residues that are necessary for desired enzymatic, immunological, or other biological activity or clearance characteristics of the protein.

In a preferred embodiment of the invention, glycoproteins are produced in cells selected for enhanced CAD activity. For example, cells are selected for enhanced CAD activity by incubation with a CAD inhibitor such as N-phosphonacetyl-L-aspartate (PALA). Cells selected for PALA resistance exhibit enhanced CAD activity.

In general, the present invention provides a process for producing glycoproteins with enhanced glycosylation comprising the step of expressing the glycoprotein in a host cell having enhanced intracellular CAD activity as compared to control host cells. In preferred embodiments, enhanced intracellular CAD activity leads to enhanced intracellular concentration of UTP which leads to improved galactosylation of recombinantly produced glycoproteins. Also described herein are host cells and cell lines with enhanced intracellular CAD as well as glycoprotein having improved galactosylation produced by expressing a heterologous nucleic acid sequence encoding the glycoprotein in a cell having enhanced CAD activity.

### BRIEF DESCRIPTION OF THE DRAWINGS

Figure 1A and 1B are schematic representations of de novo pyrimidine biosynthetic pathway.
Figure 2 is a schematic representation of the procedure used to select for CHO cells exhibiting high CAD activity using PALA.
Figure 3 is a bar graph showing CPS II activity in PALA selected CHO cells versus control cells.
Figure 4 is a gel showing CAD expression in PALA selected CHO cells.
Figure 5 shows a HPLC chromatogram of intracellular nucleotides and nucleotide sugars including UTP and UDP-Galactose in control and PALA selected CHO cells.
Figure 6 is a graph showing the stability of UTP and UDP-Galactose levels in PALA selected CHO cells over the course of 90 days.
Figure 7 is a graph showing UTP levels in CHO cells cultured in varying levels of uridine.
Figures 8A and 8B are bar graphs showing UTP (Figure 8A) and UDP-Galactose (Figure 8B) levels in CHO cells incubated with uridine; galactose; and both uridine and galactose.
Figure 9 is a schematic representation of the interplay between CAD, uridine, UTP, galactose and UDP-Galactose.
Figure 10 is a graph showing galactose content of aCD20 antibody produced in CHO cells incubated with uridine as compared with control.

### DETAILED DESCRIPTION OF THE PREFERRED EMBODIMENTS

### Definitions

The term "eukaryotic cell" or cell line is used to refer to cells established in *ex vivo* culture. It is a characteristic of the eukaryotic cell of the present invention that it expresses or is capable of expressing a particular glycoprotein of interest. Eukaryotic cells used in the production of a desired protein product have the means for glycosylating proteins by addition of oligosaccharide side chains. Such cells, in certain embodiments, also have the capability to remove and/or modify enzymatically part or all of the oligosaccharide side chains of glycoproteins.

Examples of suitable eukaryotic host cells within the context of the present invention include insect and mammalian cells. Example host cells include SF9 insect cells (Summers and Smith (1987) Texas Agriculture Experiment Station Bulletin, 1555; and Insect Cell Culture engineering, Goosen Daugulis and Faulkner Eds. Dekker, New York); Chinese hamster ovary (CHO) cells (Puck et al., (1958) J. Exp. Med. 108:945-955; Puck (1985) Molecular Cell Genetics, Gottersman MM ed. Wiley Intersciences pp37-64) including CHO cells expressing TNFR-IgG and anti-CD20; monkey kidney CV1 line transformed by SV40 (COS-7, ATCC CRL 1651); human embryonic kidney line (Graham et al., (1977) J. Gen Virol., 36:59); baby hamster kidney cells (BHK, ATCC CCL 10); and human cervical carcinoma cells (HELA, ATCC CCL 2). The above list is meant only for illustrative purposes and in no way is meant to limit the scope of the present invention.

As used herein, "control cell" refers to a cell that has been cultured in parallel with a cell treated under the specified experimental condition but unlike the treated cell, the host cell is not subjected to the specified experimental condition. Control cells represent a baseline from which comparisons are made.

The term "nucleic acid sequence" refers to the order or sequence of deoxyribonucleotides along a strand of deoxyribonucleic acid. The order of these deoxyribonucleotides determines the order of amino acids along the polypeptide chain. The deoxyribonucleotide sequence thus codes for the amino acid seqeunce.

The term "expression vector" refers to a piece of DNA, usually doublestranded, which may have inserted into it a piece of DNA, for example a piece of foreign DNA. Foreign DNA is defined as heterologous DNA, which is DNA not naturally found in the host cell and includes additional copies of genes naturally present in the host genome. The vector is used to transport the foreign or heterologous DNA into a suitable host cell. Once in the host cell, the vector is capable of integration into the host cell chromosomes. The vector contains the necessary elements to select cells containing the integrated DNA as well as elements to promote transcription of polyadenylated messenger RNA (mRNA) from the transfected DNA. Many molecules of the polypeptide encoded by the foreign DNA can thus be rapidly synthesized.

The term "expression" or "expresses" are used herein to refer to transcription and translation occurring within a host cell. The level of expression of a product gene in a host cell may be determined on the basis of either the amount of corresponding mRNA that is present in the cell or the amount of the protein encoded by the product gene that is produced by the cell. For example, mRNA transcribed from a product gene is desirably quantitated by northern hybridization. Sambrook *et al.,* Molecular Cloning: A Laboratory Manual, pp. 7.3-7.57 (Cold Spring Harbor Laboratory Press, 1989). Protein encoded by a product gene can be quantitated either by assaying for the biological activity of the protein or by employing assays that are independent of such activity, such as western blotting or immunoassay using antibodies that are capable of reacting with the protein. Sambrook *et al.,* Molecular Cloning: A Laboratory Manual, pp. 18.1-18.88 (Cold Spring Harbor Laboratory Press, 1989).

As used herein, "glycoprotein" refers generally to peptides and proteins having more than about 10 amino acids and at least one carbohydrate. The glycoproteins may be homologous to the host cell, or preferably, be heterologous, i.e., foreign, to the host cell. Example glycoproteins that could be expressed using one embodiment of the present invention include molecules such as cytokines and their receptors, chimeric proteins, tumor necrosis factor alpha and beta, tumor necrosis factor receptors, human growth hormone, bovine growth hormone, parathyroid hormone, thyroid stimulating hormone, lipoproteins, alpha-1-antitrypsin, insulin A-chain, T-cell receptors, surface membrane proteins, monoclonal antibodies, and transport proteins. The list of examples is illustrative only and in no way is meant to limit the scope of the present invention.

The term "glycoform" as used within the context of the present invention is meant to denote a glycoprotein containing a particular carbohydrate structure or structures.

The term "glycosylation" as used within the context of the present invention is meant to encompass the process by which a protein is covalently linked with one or more oligosaccharide chains. The term "galactosylation" means the addition of a galactose units to an oligosaccharide chain on a glycoprotein and "sialylation" the addition of a sialic acid to an oligosaccharide chain on a glycoprotein.

"Period of time and under such conditions that cell growth in maximized", and the like, refer to those culture conditions that, for a particular cell line , are determined to be optimal for cell growth and division. Normally, during cell culture cells are cultured in nutrient medium containing the necessary additives generally at about 30-40° C, in a humidified, controlled atmosphere, such that optimal growth is achieved for that particular cell line.

As used herein "cultured for sufficient time to allow selection to occur" refers to the act of physically culturing the eukaryotic host cells on the selection agent until resistant clones were picked and tested for the relevant characteristic.

As used herein, protein, peptide and polypeptide are used interchangeably to denote an amino acid polymer or a set of two or more interacting or bound amino acid polymers.

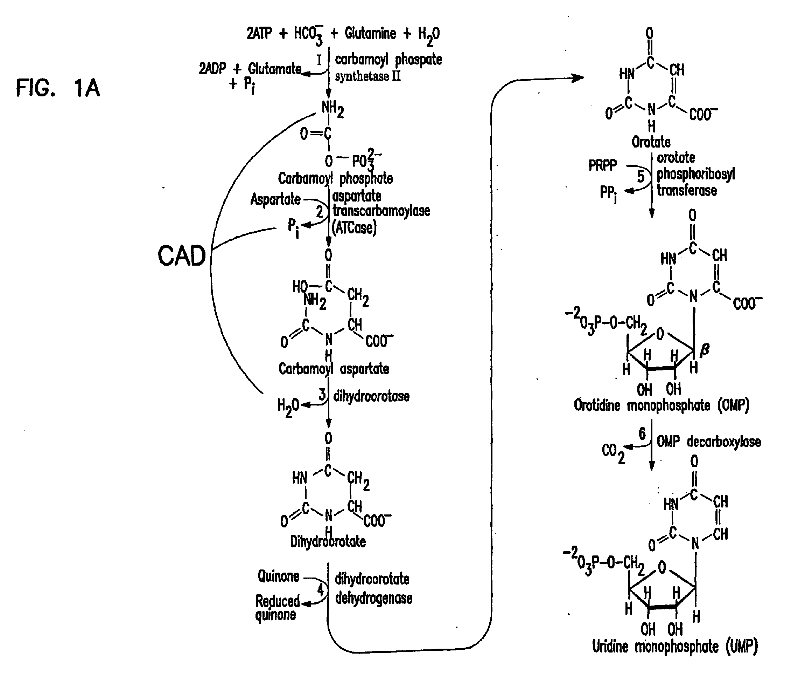

As used herein, "CAD" refers to the multienzyme polypeptide complex (carbamoyl phosphate synthetase (CPS II), aspartate transcarbamoylase and dihydro-orotase) that catalyzes the first three reactions in the *de novo* biosynthesis of pyrimidines. (see Figure 1A). The rate limiting step for pyrimidine biosynthesis is the synthesis of carbamoyl phosphate from bicarbonate, ATP, and glutamine by CPS II (one of the enzyme activities associated with CAD). (Irvine et al (1997) Eur. J. Biochem 247:1063-1073) Ultimately CAD, in association with two other enzymes, orotate phosphoribosyl transferase and orotidylate decarboxylase, yield UMP, which is ultimately converted to UTP.

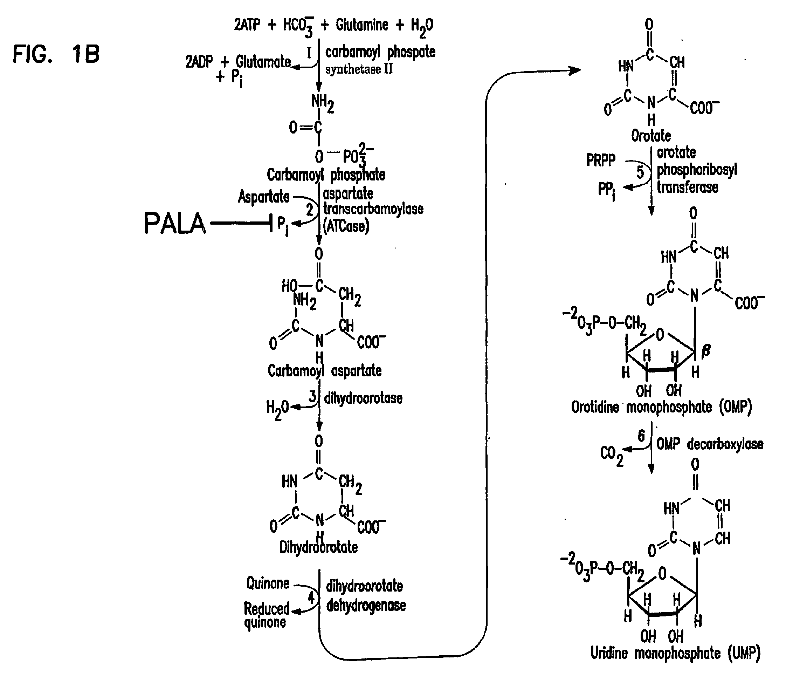

As used herein, CAD inhibitors include any substance known to inhibit the activities of the CAD enzyme complex. CAD inhibitors include N-(phosphoacetyl)-L-aspartate (see Figure 1B).

### Modes For Carrying Out the Invention

The present invention is based upon, among other things, the unexpected discovery that production of glycoproteins in host cell lines generally results in under-glycosylated oligosaccharide glycoprotein profiles. According to the present invention, the expression of glycoproteins in a host cell having enhanced CAD activity, where the host cell is capable of expressing a glycoprotein of interest, results in consistently enhanced oligosaccharide glycoprotein profiles.

### Enhanced CAD Activity

Selection of enhanced CAD activity within a host cell expressing or capable of expressing a glycoprotein of interest may occur by any means known in the art. In a particular embodiment, cells may be treated with a CAD inhibitor and the surviving, resistant cells selected for enhanced CAD activity. The CAD inhibitor may be any known to the art. One preferred CAD inhibitor for use in the present invention is P-(phosphonacetyl)-L-Aspartate (PALA). PALA treated cells are typically selected using final concentrations of PALA between 2 to 10 mM.

A cell, preferably a mammalian cell or insect cell, most preferably a chinese hamster ovary (CHO) cell, such as CHO DP12, CHO DG44 or CHO Kl, is selected under the pressure of a CAD inhibiting factor such as PALA. Cells are cultured in a basal medium such as DMEM/HAM F-12 based formulation (for composition of DMEM and HAM F12 media, see culture media formulations in American Type Culture Collection Catalogue of Cell Lines and Hybridomas, Sixth Edition, 1988, pages 346-349) (the formulation of medium as described in U.S. Patent 5,122,469 are particularly appropriate) having the appropriate CAD inhibitor, with modified concentrations of some components such as amino acids, salts, sugar, and vitamins, and optionally containing glycine, hypoxanthine, and thymidine; recombinant human insulin, hydrolyzed peptone, such as Primatone HS or Primatone RL (Sheffield, England), or the equivalent; a cell protective agent, such as Pluronic F68 or the equivalent pluronic polyol; Gentamycin; and trace elements.

Surviving cells, grown under CAD inhibitor selection, *i.e.,* CAD inhibitor resistant cells, are tested for CAD activity by any number of known assays within the art. Examples include, the ammonia-dependent part reaction (Shaw et al (Shaw et al (1992) Eur. J. Biochem. 207:957-965) and the radiometric procedure (Guy et al (Guy et al (1997) JBC 272(46):29255-29262).

Enhanced intracellular CAD activity can also be provided by over-expressing CAD within a cell. Human CAD has been cloned (Iwahana et al., (1996) Biochemical and Biophysical Research Comm., 219:249-255) as has the enzymatic domains of hamster CAD. Davidson et al., (1995) Plenum Press, New York, 591-595. Over-expression can be of the entire CAD protein or any part thereof that provides sufficient CAD activity for the purposes of the present invention. Well known procedures for introducing nucleic acid sequences encoding CAD into the cell may be used. These include plasmid vectors, viral vectors, and other methods for introducing genetic material into a host cell. It is necessary that the CAD gene be introduced in such a way that the host cell expresses the protein. High level expression of CAD is preferred. The techniques for over-expressing a protein within a host cell are discussed in greater detail below in the context of glycoprotein production.

Another suitable method for enhancing intracellular CAD activity is the expression of mutated CAD, where the mutation reduces inhibitory regulation by the enzyme's products, e.g., by UTP or UTP analogs. Nucleic acid sequences having one or more mutation in the UTP binding site of CAD are useful, although the mutation may occur anywhere in the nucleic acid sequence sufficient to disrupt UTP regulation of the encoded mutant CAD protein.

### Increased UTP and UDP-Galactose Levels

Enhanced intracellular CAD activity leads to increased intracellular UTP and UDP-galactose concentrations. Cells exhibiting enhanced CAD activity are analyzed for UTP and UDP-galactose levels by suitable methods know within the art such as the method of Ryll and Wagner (1991) J. Chromatography, 570:77-88.

### Glycoprotein

According to the invention, host cells resistant to CAD inhibitor, such as those described above, are modified to functionally express an endogenous glycoprotein or a recombinant glycoprotein. One preferred embodiment of the present invention provides PALA resistant CHO cells, employed for high yield expression of glycoproteins from engineered vectors. The glycoprotein expressed by the CHO cell is generally produced from DNA transfected into the cell. The structure and extent of the carbohydrate portion of the glycoprotein is determined by the cellular machinery of the host cell, in this example, the PALA resistant CHO cell.

Nucleic acid sequences encoding an endogenous host cell protein or a heterologous recombinant glycoprotein are available to the skilled artisan and may be obtained, for example by *in vitro* synthesis methods or obtained readily from cDNA libraries. The means for synthetic creation of the DNA, either by hand or with an automated apparatus, are generally known to one of ordinary skill in the art. Examples of the current techniques available for polynucleotide synthesis are found, for example, in Maniatis et al., Molecular Cloning-A Laboratory Manual, Cold Spring Harbor Laboratory (1984), and Horvath et al., An Automated DNA Synthesizer Employing Deoxynucleotide 3'-Phosphoramidites, Methods in Enzymology 154: 313-326, 1987, hereby specifically incorporated by reference. Alternatively, the gene sequence encoding the recombinant glycoprotein is cloned from cDNA libraries employing techniques available to the skilled artisan. For example, polymerase chain reaction techniques may be employed whereby a particular nucleic acid sequence is amplified. Oligonucleotide primers based upon sequences which correspond to the 3' and 5' ends of the segment of the DNA to be amplified are hybridized under appropriate conditions and the enzyme Taq polymerase, or equivalent enzyme, is used to synthesize copies of the DNA located between the primers.

The particular procedure used for the functional expression of the recombinant glycoprotein is not critical to the invention. For example, any procedure for introducing nucleotide sequences into host cells may be used. These include the use of plasmid vectors, viral vectors, and other methods for introducing genetic material into a host cell. It is necessary that the gene or nucleic acid to be expressed be introduced in such a way that the host cell expresses the protein. High level expression is preferred.

For example, expression is typically achieved by introducing into the cells the appropriate glycoprotein along with another gene, commonly referred to as a selectable gene, that encodes a selectable marker. A selectable marker is a protein that is necessary for the growth or survival of a host cell under the particular culture conditions chosen, such as an enzyme that confers resistance to an antibiotic or other drug, or an enzyme that compensates for a metabolic or catabolic defect in the host cell. For example, selectable genes commonly used with eukaryotic cells include the genes for aminoglycoside phosphotransferase (APH), hygromycin phosphotransferase (hyg), dihydrofolate reductase (DHFR), thymidine kinase (tk), neomycin resistance, puromycin resistance, glutamine synthetase, and asparagine synthetase. In selecting an appropriate expression system, a selectable marker for the glycoprotein is chosen to allow for, if necessary, a second transfection with a second suitable amplifiable marker for the expression of a second glycoprotein product or to allow additional functional modifications of the host cell. Such modifications may include augmentation of other activities related to improving cell metabolism of the quantity or quality of recombinant product expression.

The level of expression of a gene introduced into a eukaryotic host cell of the invention depends on multiple factors, including gene copy number, efficiency of transcription, messenger RNA (mRNA) processing, stability, and translation efficiency. Accordingly, high level expression of a desired glycoprotein according to the present invention will typically involve optimizing one or more of those factors.

Further, the level of glycoprotein production may be increased by covalently joining the coding sequence of the gene to a "strong" promoter or enhancer that will give high levels of transcription. Promoters and enhancers that interact specifically with proteins in a PALA resistant host cell that are involved in transcription are suitable within the context of the present invention. Among the eukaryotic promoters that have been identified as strong promoters for high-level expression which are preferred within the context of the present invention are the SV40 early promoter, adenovirus major late promoter, mouse metallothionein-I promoter, Rous sarcoma virus long terminal repeat, and human cytomegalovirus immediate early promoter (CMV). Particularly useful in expression of the desired glycoprotein product are strong viral promoters such as the myeloproliferative sarcoma virus (Artel et al., (1988) Gene 68:213-220), SV40 early promoter (McKnight and Tijian (1986) Cell, 46:795-805).

Enhancers that stimulate transcription from a linked promoter are also useful in the context of the present invention. Unlike promoters, enhancers are active when placed downstream from the transcription initiation site or at considerable distances from the promoter, although in practice enhancers may overlap physically and functionally with promoters. For example, many of the strong promoters listed above also contain strong enhancers (Bendig, (1988) Genetic Engineering, 7:91).

The level of protein production or expression also may be increased by increasing the gene copy number in the host cell. One method for obtaining high gene copy number is to directly introduce into the host cell multiple copies of the gene, for example, by using a large molar excess of the product gene relative to the selectable gene during cotransfectation. Kaufman, (1990) Meth. Enzymol., 185:537. With this method, however, only a small proportion of the cotransfected cells will contain the product gene at high copy number. Screening methods typically are required to identify the desired high-copy number transfectants.

The use of splice-donor style vectors such as those described by Lucas et al., (1996) Nuc. Acd Res. 24(9):1774-1779 and WO/9604391 is also preferred.

Yet another method for obtaining high gene copy number involves gene amplification in the host cell. Gene amplification occurs naturally in eukaryotic cells at a relatively low frequency (Schimke,(1988) J. Biol. Chem., 263:5989). However, gene amplification also may be induced, or at least selected for, by exposing host cells to appropriate selective pressure. For example, in many cases it is possible to introduce a glycoprotein gene together with an amplifiable gene into a host cell and subsequently select for amplification of the marker gene by exposing the cotransfected cells to sequentially increasing concentrations of a selective agent. Typically the product gene will be coamplified with the marker gene under such conditions.

The most widely used amplifiable gene for that purpose is a DHFR gene, which encodes a dihydrofolate reductase enzyme. The selection agent used in conjunction with a DHFR gene is methotrexate (MTX). In this example, a PALA resistant cell is cotransfected with the glycoprotein gene and a DHFR gene, and transfectants are identified by first culturing the cells in culture medium that contains MTX. The transfected cells then are exposed to successively higher amounts of MTX. This leads to the synthesis of multiple copies of the DHFR gene, and concomitantly, multiple copies of the glycoprotein gene (Schimke, (1988) J. Biol. Chem., 263:5989; Axel et al., U.S. Patent No. 4,399,216; Axel et al., U.S. Patent No. 4,634,665; Kaufman in Genetic Engineering, ed. J. Setlow (Plenum Press, New York), Vol. 9 (1987); Kaufinan and Sharp, (182) J. Mol. Biol., 159:601; Ringold et al., J. Mol. Appl. Genet., 1:165-175; Kaufman et al., Mol. Cell Biol., 5:1750-1759; Kaetzel and Nilson, (1988) J. Biol. Chem., 263:6244-6251; Hung et al., (1986) Proc. Natl. Acad. Sci. USA, 83:261-264; Kaufman et al., (1987) EMBO J., 6:87-93; Johnston and Kucey, (1988) Science, 242:1551-1554; Urlaub et al., (1983) Cell, 33:405-412.

Alternatively, host cells may be co-transfected with a glycoprotein gene, a DHFR gene, and a dominant selectable gene, such as a neo^{r} gene. Transfectants are identified by first culturing the cells in culture medium containing neomycin (or the related drug G418), and the transfectants so identified then are selected for amplification of the DHFR gene and the product gene by exposure to successively increasing amounts of MTX.

Another method involves the use of polycistronic mRNA expression vectors containing a product gene at the 5' end of the transcribed region and a selectable gene at the 3' end. Because translation of the selectable gene at the 3' end of the polycistronic mRNA is inefficient, such vectors exhibit preferential translation of the glycoprotein gene and require high levels of polycistronic mRNA to survive selection. Kaufman, (1990) Meth. Enzymol., 185:487; Kaufman, (1990) Meth. Enzymol., 185:537; Kaufman et al., (1987) EMBO J., 6:187. Accordingly, cells expressing high levels of the desired protein product may be obtained in a single step by culturing the initial transfectants in medium containing a selection agent appropriate for use with the particular selectable gene.

A further method suitable within the context of the present invention is integrate the genes encoding the glycoprotein into a transcriptionally active part of the PALA resistant cell genome. Such procedures are described in International Application No. PCT/US/04469.

Other mammalian expression vectors such as those that have single transcription units are also useful in the context of the present invention. Retroviral vectors have been constructed (Cepko et al., (1984) Cell, 37:1053-1062) in which a cDNA is inserted between the endogenous Moloney murine leukemia virus (M-MuLV) splice donor and splice acceptor sites which are followed by a neomycin resistance gene. This vector has been used to express a variety of gene products following retroviral infection of several cell types.

Introduction of the nucleic acids encoding the glycoprotein is accomplished by methods known to those skilled in the art. For mammalian cells without cell walls, the calcium phosphate precipitation method of Graham and van der Eb, (1978) Virology, 52:456-457 may be used. General aspects of mammalian cell host system transformations have been described by Axel in U.S. 4,399,216 issued 16 August 1983. However, other methods for introducing DNA into cells such as by nuclear injection, by protoplast fusion or by electroporation may also be used (Chisholm et al., (1995) DNA Cloning IV: A Practical Approach, Mammalian Systems., Glover and Hanes, eds., pp. 1-41). In the preferred embodiment the DNA is introduced into the host cells using lipofection. See, Andreason, (1993) J. Tiss. Cult. Meth., 15:56-62, for a review of electroporation techniques useful for practicing the instantly claimed invention.

Gene amplification and/or expression may be measured in a sample directly, for example, by conventional Southern blotting, Northern blotting to quantitate the transcription of mRNA (Thomas, (1980) Proc. Natl. Acad. Sci. USA, 77:5201-5205), dot blotting (DNA or RNA analysis), RT-PCR or *in situ* hybridization, using an appropriately labeled probe, based on the sequences provided herein. Various labels may be employed in constructing probes, most commonly radioisotopes, particularly ³²P. However, other techniques may also be employed, such as using biotin-modified nucleotides for introduction into a polynucleotide. The biotin then serves as the site for binding to avidin or antibodies, which may be labeled with a wide variety of labels, such as radionuclides, fluorescens, enzymes, or the like. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

For the culture of the PALA resistant cells expressing the desired protein and modified as described for the instant invention, numerous culture conditions can be used paying particular attention to the resistant cell being cultured. Suitable culture conditions for eukaryotic cells are well known in the art (J. Immunol. Methods (1983)56:221-234) or can be easily determined by the skilled artisan (see, for example, *Animal Cell Culture: A Practical Approach,* 2nd Ed., Rickwood, D. and Hames, B.D., eds. Oxford University Press, New York (1992)), and vary according to the particular cell selected.

For the production of the sought after glycoproteins, production by growing the host cells of the present invention under a variety of cell culture conditions is typical. For instance, cell culture procedures for the large or small scale production of proteins are potentially useful within the context of the present invention. Procedures including, but not limited to, a fluidized bed bioreactor, hollow fiber bioreactor, roller bottle culture, or stirred tank bioreactor system may be used, in the later two systems, with or without microcarriers, and operated alternatively in a batch, fed-batch, or continuous mode.

Following the glycoprotein production phase, the polypeptide of interest is recovered from the culture medium using techniques which are well established in the art.

The polypeptide of interest preferably is recovered from the culture medium as a secreted polypeptide, although it also may be recovered from cell lysates.

As a first step, the culture medium or lysate is centrifuged to remove particulate cell debris. The polypeptide thereafter is purified from contaminant soluble proteins and polypeptides, with the following procedures being exemplary of suitable purification procedures: by fractionation on immunoaffinity or ionexchange columns; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG. A protease inhibitor such as phenyl methyl sulfonyl fluoride (PMSF) also may be useful to inhibit proteolytic degradation during purification.

### Glycosylation of Glycoprotein

The complex carbohydrate portion of the glycoprotein produced by the processes of the present invention may be readily analyzed if desired, by conventional techniques of carbohydrate analysis to confirm the oligosaccharide content of the glycoprotein. Thus, for example, techniques such as lectin blotting or monosaccharide analysis, well-known in the art, reveal proportions of terminal mannose, N-acetylglucosamine, sialic acid or other sugars such as galactose.

The resulting carbohydrates can be analyzed by any method known in the art including those methods described herein. Several methods are known in the art for glycosylation analysis and are useful in the context of the present invention. Such methods provide information regarding the identity and the composition of the oligosaccharide attached to the peptide. Methods for carbohydrate analysis useful in the present invention include but are not limited to capillary electrophoresis (Rassi and Nashabeh, High Performance Capillary Electrophoresis of Carbohydrates and Glycoconjugates, In, Carbohydrate Analysis (Z. El Rassi, ed.) (1995) 58:267-360), fluorophore-assisted carbohydrate electrophoreses (Starr et al., (1996) J. Chrom. A 720:295-321), high pH anion exchange chromatography pulsed amperometric detection (HPAEC PAD) (Lee (1996) J. Chrom. A 720:137-151), matrix assisted laser desorption/ionization time of fight mass spectrometry (Harvey (1996) J. Chrom. A 720:429-447; Papac et al., (1996) Anal. Chem. 68:3215-3223; Field et al. (1996) Anal. Biochem. 239:92-98; Hooker et al., (1995) Biotechnology and Bioeng. 48:639-648), high performance liquid chromatogrphy (El Rassi (1995) Reversed phase and hydrophobic interaction chromatogrphy of carbohydrates and glycoconjugates. In, Carbohydrate Analysis (Z. El Rassi, ed.) 58:267-360), and electrospray ionization mass spectrometry Roberts G.D. (1995) Anal. Chem. 67:3613-3625).

Neutral and amino-sugars can be determined by high performance anion-exchange chromatography combined with pulsed amperometric detection (HPAE-PAD Carbohydrate System, Dionex Corp.). For instance, sugars can be released by hydrolysis in 20% (v/v) trifluoroacetic acid at 100EC for 6 h. Hydrolysates are then dried by lyophilization or with a Speed-Vac (Savant Instruments). Residues are then dissolved in 1 % sodium acetate trihydrate solution and analyzed on a HPLC-AS6 column as described by Anumula *et al.* (Anal. Biochem. 195:269-280 (1991).

Alternatively, immunoblot carbohydrate analysis may be performed. According to this procedure protein-bound carbohydrates are detected using a commercial glycan detection system (Boehringer) which is based on the oxidative immunoblot procedure described by Haselbeck and Hosel (Haselbeck et al. (1990) Glycoconjugate J., 7:63). The staining protocol recommended by the manufacturer is followed except that the protein is transferred to a polyvinylidene difluoride membrane instead of nitrocellulose membrane and the blocking buffers contained 5% bovine serum albumin in 10 mM tris buffer, pH 7.4 with 0.9 % sodium chloride. Detection is made with anti-digoxigenin antibodies linked with an alkaline phosphate conjugate (Boehringer), 1:1000 dilution in tris buffered saline using the phosphatase substrates, 4-nitroblue tetrazolium chloride, 0.03% (w/v) and 5-bromo-4 chloro-3-indoyl-phosphate 0.03 % (w/v) in 100 mM tris buffer, pH 9.5, containing 100 mM sodium chloride and 50 mM magnesium chloride. The protein bands containing carbohydrate are usually visualized in about 10 to 15 min.

The carbohydrate may also be analyzed by digestion with peptide-N-glycosidase F. According to this procedure the residue is suspended in 14 F1 of a buffer containing 0.18% SDS, 18 mM beta-mercaptoethanol, 90 mM phosphate, 3.6 mM EDTA, at pH 8.6, and heated at 100 EC for 3 min. After cooling to room temperature, the sample is divided into two equal parts. One aliquot is not treated further and serves as a control. The second fraction is adjusted to about 1% NP-40 detergent followed by 0.2 units of peptide-N-glycosidase F (Boehringer). Both samples are warmed at 37° C for 2 hr and then analyzed by SDS-polyacrylamide gel electrophoresis.

### EXAMPLES

The following examples are provided to illustrate the invention only, and should not be construed as limiting the scope of the invention.

### Example 1

### PALA Resistant CHO Cells Express Increased Carbamoylphosphate Synthetase II Activity

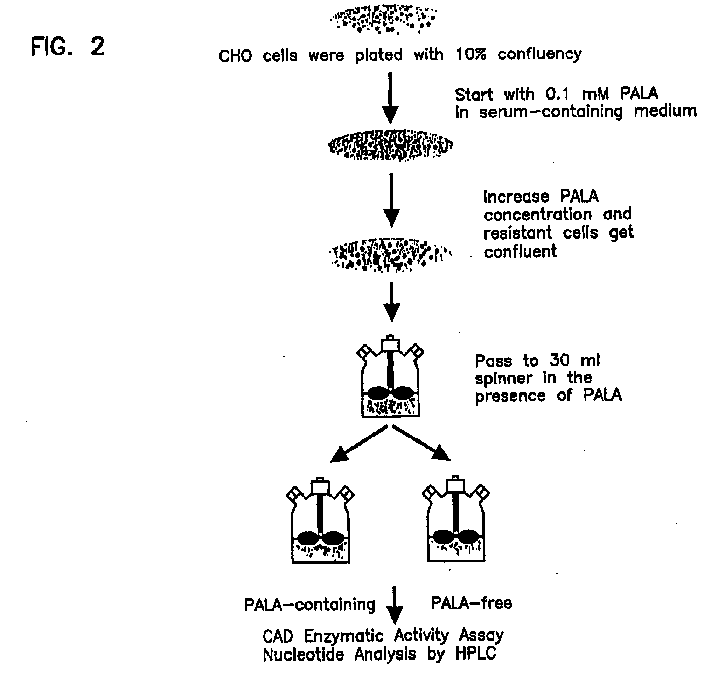

CHO DP12 and CHO DG44 parental cell lines and CHO DP12 expressing TNFR-IgG and anti-CD11a were cultivated with and without P-phosphonacytyl-L-aspartate (PALA). As shown schematically in Figure 2, each cell line was plated at 10% confluency in 10cm petri dishes using standard tissue culture techniques. Cells were cultured in serum containing medium having 0.1mM PALA. The culture medium was changed weekly. Surviving clones were pooled and sub-cultivated in increasing concentrations of PALA until the target PALA concentration was reached, i.e., 2-10 mM PALA. After selection for the PALA phenotype (see below), cells were either passed into a PALA containing medium or to a PALA free medium and further cultivated in suspension spinner flasks. Spinner flask cell populations were sub-cultivated every 4-7 days. Cells selected in the presence of PALA are referred to as PALA-resistant cells, cells cultivated in the same manner as above but not treated with PALA will be referred to as control cells.

CHO cells treated with 3mM PALA were analyzed for CPS II activity, an indicator of overall CAD activity, using the method generally described in Mori and Tatibana. Mori and Tatibana (1978) Methods in Enzymology, 51: 111-112. Two PALA resistant CHO sub-cultures were analyzed, CHO-A and CHO-B, to ensure that results were uniform to the PALA resistant phenotype and not particular to a sub-population of treated cells.

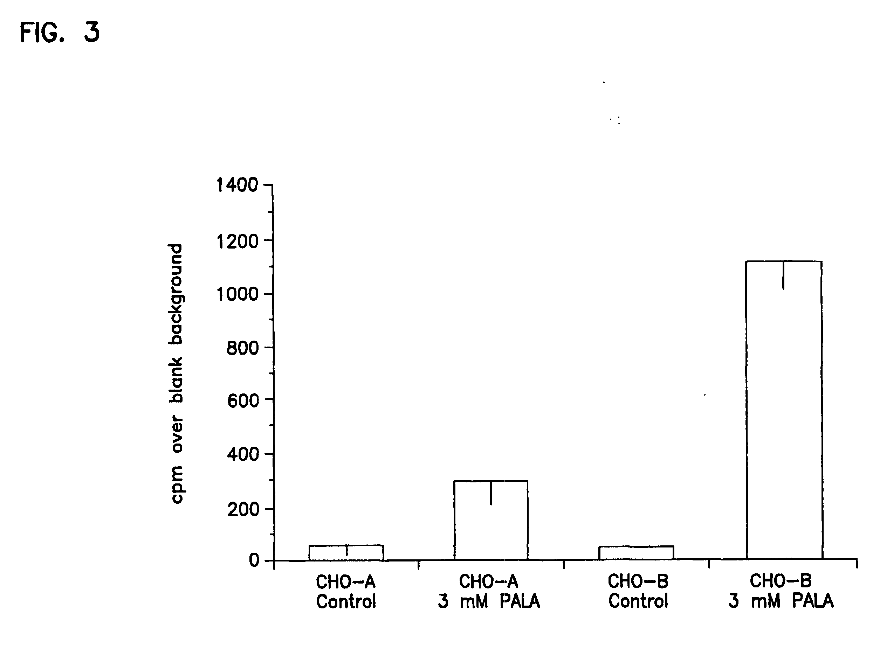

The data are shown in Figure 3. PALA resistant cells demonstrated increased CPS II activity over CHO control cells. In particular, the CHO B cell line showed an approximate twenty fold increase in CPS II activity as compared to control CHO cells.

The data demonstrates that PALA resistant CHO cells have increased CPS II activity, and thus increased CAD activity, as compared to non-treated control cells.

### Example II

### PALA Resistant CHO Cells Having Increased CAD Expression

The PALA resistant cells obtained in Example 1 were used to determine if increased CPS II activity exhibited in PALA resistant cells was a result of increased CAD expression in those cells. CAD expression was measured on PALA resistant cells using a RNase protection assay similar to the method described in Mahler and McGuire. Mahler and McGuire (1990), J. Clin. Invest., 86: 1641-1648. In particular, TRY cells were selected under 2 mM PALA and CAD mRNA expression measured using a RNase protection assay.

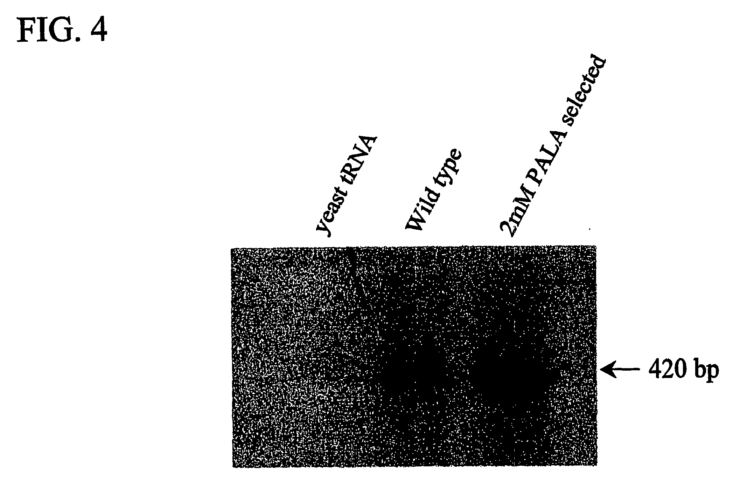

The data are shown in Figure 4, and demonstrate that increased CPS II activity in PALA resistant cells corresponds to increased CAD expression within those cells. More than a two fold increase in CAD mRNA is observed in PALA selected cells as compared to control cells.

### Example III

### Increased Intracellular UTP in Cells Having Increased CAD Activity

The PALA resistant cells of Example 1 were used to determine if PALA resistant cells showed increased expression of UTP.

CHO cells were treated in 5 mM PALA, cell extracts were prepared and nucleotides measured using a method similar to Ryll and Wagner. Ryll and Wagner (1991) J. Chromatography, 570:77-88. The experiment was performed over a time course of 10 to 90 days and data quantified and plotted as UTP content of PALA resistant cells relative to control cells.

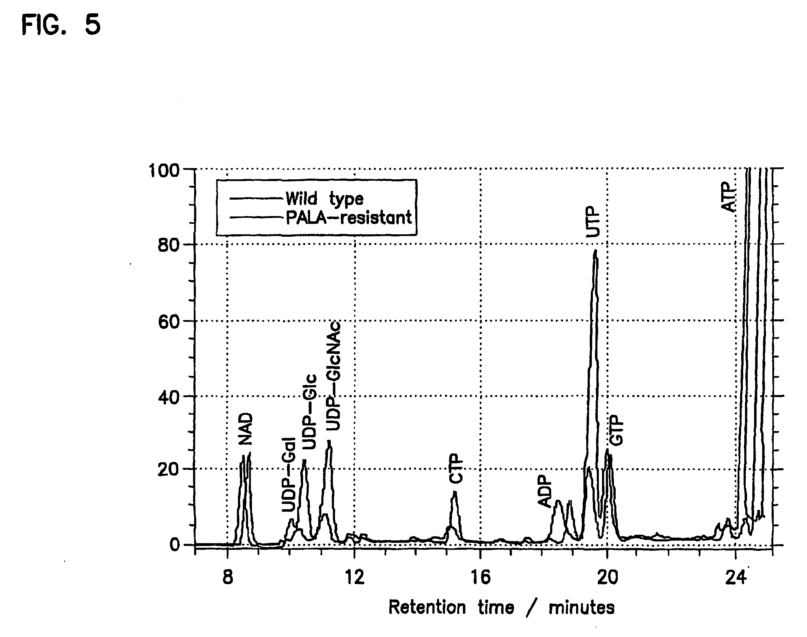

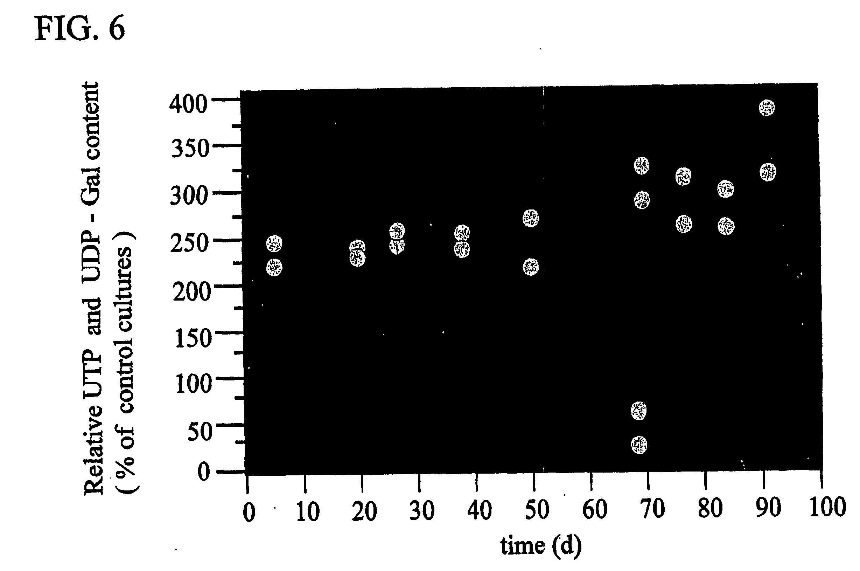

As shown in Figure 5, PALA resistant cells having increased CAD activity showed markedly increased UTP elution peaks as compared to the UTP elution peak for control cells. Figure 6 shows increased UTP concentrations in the PALA resistant cells were stable over a period of at least 90 days. PALA resistant cells showed an approximate 200-350 % increase in UTP content as compared to control cells during the entire period of the experiment.

The data demonstrates that cells having increased CAD activity also have increased UTP levels. CPS II activity is known to be negatively inhibited by UTP. (Carry, (1985) EMBO J. 4:3735-3742); (Chernova et al (1998) MCB 18:536-545) Therefore, at high intracellular UTP concentrations, CPS II activity is expected to be low (inhibited). Similarly, at low intracellular UTP levels, CPS II activity is expected to be high (uninhibited). Surprisingly, as shown in Figures 5 and 6, this was not the situation in cells selected for enhanced CAD activity. Cells having elevated CAD activity also demonstrated elevated intracellular UTP levels. This high UTP phenotype was fairly stable, as the cells having enhanced CAD activity showed enhanced levels of UTP for at least 90 days.

### Example IV

### Cells Having Increased CAD Activity Have Increased UDP-Galactose Levels And Presumably Increased Glycosylation

The PALA resistant cells and methods of Example 1 were used to determine if PALA resistant cells showed increased expression of UDP-galactose. Further, PALA resistant CHO DP12 cells expressing TNFR-IgG were seeded at 4x10⁶ cells per ml in spinner flask cultures and incubated at 31°C and 6 mM butyrate for 6 days. The cell culture supernatant was harvested and TNFR-IgG recovered by a method of protein A immunoaffinity chromatography as is well known in the art. N-linked glycans were analyzed by MALDI mass spectrometry using the method generally described by Papac et al., (1996) Anal. Chem., 68:3215-3223. Sialic acid content of the TNFR-IgG was determined using were analyzed using the method generally described by Anumula, (1995) Anal. Biochem., 230, 24-30.

The UDP-galactose data are shown in Figures 5 and 6. In Figure 5, PALA resistant cells having increased CAD activity showed markedly increased UDP-galactose elution peaks as compared to the UDP-galactose elution peak for control cells. In Figure 6, increased UDP-galactose concentrations in the PALA resistant cells were stable over a period of at least 90 days. PALA resistant cells showed an approximate 200-350 % increase in UDP-galactose content as compared to control cells during the entire period of the experiment. It is further expected, that both the N-linked glycans and the sialic acid content of TNFR-IgG from cells having enhanced CAD activity will be increased as compared to control cells.

The data demonstrates that PALA-resistant cells having increased CAD activity also have increased intracellular UDP-galactose levels. The percent increase is similar to the increases shown in Example III above for UTP. Thus, like UTP, UDP-galactose concentrations are markedly increased in cells having increased CAD activity. Because feeding studies have demonstrated that enhanced intracellular UDP-galactose concentrations correlate with enhanced protein glycosylation, the present data suggests that the enhanced CAD activity and enhanced intracellular UDP-galactose in the selected cells will result in increased glycosylation of protein produced in the cells. UDP-galactose is a known substrate in the glycosylation pathway.

### Example V

### Cells Fed Uridine Have Increased UTP Levels

CHO cells were incubated under standard tissue culture technique in the presence and absence of 1 to 20 mM uridine for 18 hours and tested for UTP concentrations using a method similar to Ryll and Wagner. Quantified data from the 0, 1, 2, 5, 10 and 20 mM uridine cultures were plotted as a function of normalized intracellular UTP concentrations. Alternatively, CHO Cells were cultured in the presence of 0.2 mM uridine or 11 mM galactose and tested for UTP concentrations using the method of Ryll and Wagner.

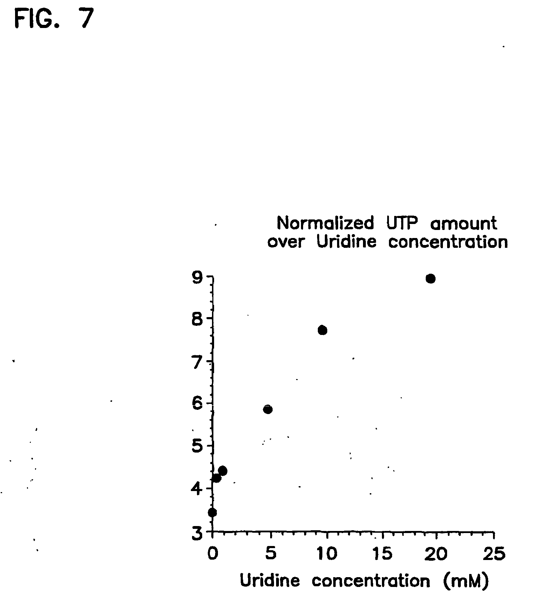

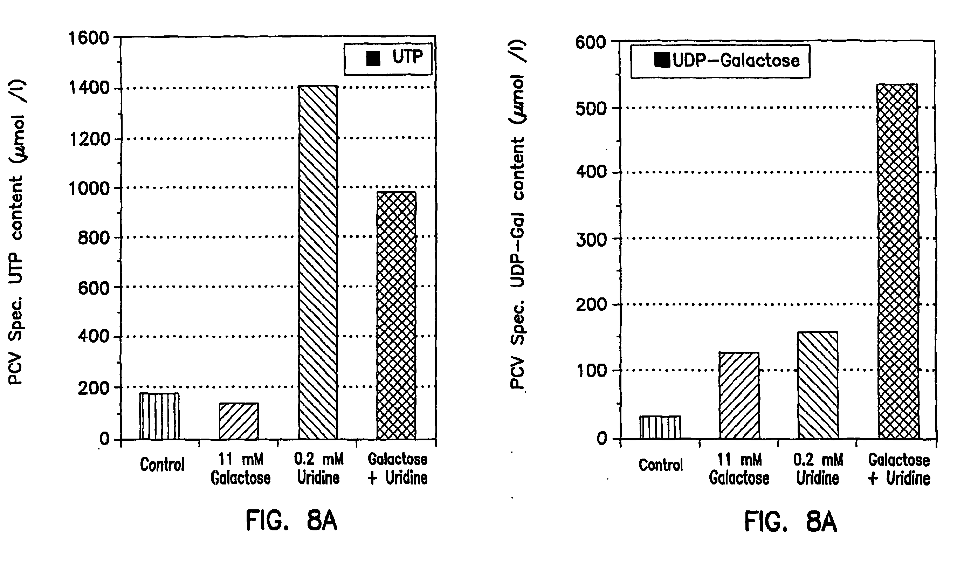

The data are shown in Figures 7 and 8. In Figure 7, the data demonstrates that feeding the CHO cells uridine increases UTP levels within the cell in a dose dependent manner. In Figure 8, the data confirms that feeding CHO cells uridine leads to a dramatic increase in UTP levels within the cell. Further, in Figure 8, the data demonstrates that feeding cells galactose, unlike uridine, does not increase UTP levels within the cell but does increase UDP-galactose levels in the cells.

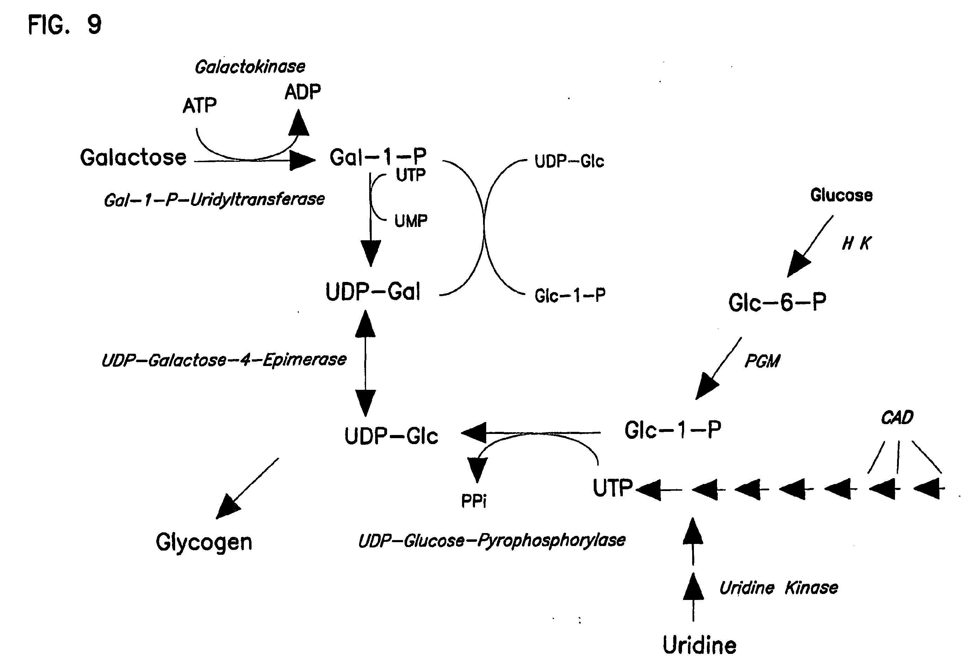

The data demonstrates that incubation of CHO cells in uridine leads to an increase in UTP levels within the cell. This increase in UTP levels was the result of uridine treatment, as treatment with galactose did not effect cellular UTP levels (this is the expected result because galactose is not a substrate for UTP production - see Figure 9).

### Example VI

### Cells Fed Uridine Show Increased Galactosylation

A CHO cell line that produces a recombinant glycoprotein, monoclonal antibody (MAb) aCD20, was cultured in the presence and absence of 0.5 to 1.0 mM uridine for a period of 7 days. Secreted aCD20 MAb was purified by immunoaffinity using the method of Ma & Nashabeh. Ma & Mashabeh (1999) Analytical Chemistry 71(22):5185-92. Purified aCD20 was tested for galactose content using the capillary electrophoresis method of Ma & Nashabeh and plotted as a percent galactose content.

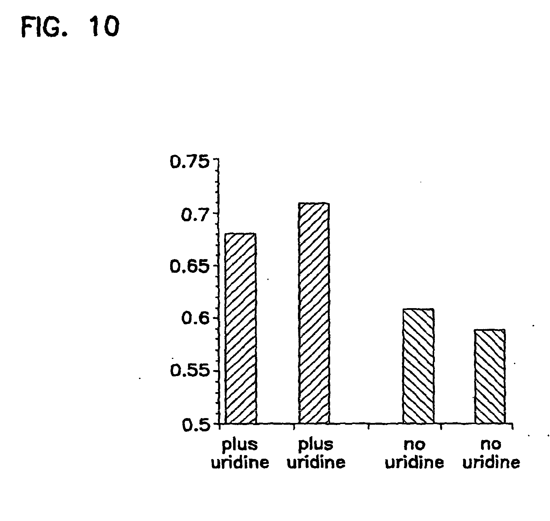

The data are shown in Figure 10. In Figure 10, the aCD20 MAb from uridine treated cells had a much higher galactose content than did the aCD20 from non-uridine treated control cells.

The data demonstrates that cells having increased UTP concentrations show increased galactosylation of protein produced in the cells.

These example taken together demonstrate that cells expressing enhanced levels of CAD are excellent hosts for the expression of endogenous and heterologous glycoprotein production. Cells having enhanced CAD activity have increased UTP pools and are thus able to support enhanced glycosylation. Cells able to support increased glycosylation are critical to producing glycoproteins having enhanced oligosaccharide content.

### REFERENCES

Anumula, K.R. (1995)
   Rapid Quantitative Determination of Sialic Acid in Glycoproteins by High-Performance Liquid Chromatography with a Sensitive Fluorescence Detection. Anal. Biochem., 230:24-30.
Papac, D, Wong, A, Jones A. (1996)
   Analysis of acidic oligosaccharides and glycoproteins by matrix-assisted laser desportion/ionization time-of-flight mass spectrometry. Anal. Chem., 68:3215-3223.
Ryll, T, Wagner, R. (1991)
   Improved ion-pair high-performance liquid chromatographic method for the quantification of a wide variety of nucleotides and sugar nucleotides in animal cells. J. Chromatography, 570:77-88.
**CAD Cloning**
   Iwahana, H, Fujimura, M, Ii, S, Kondo, M, Moritani M., Takahashi, Y., Yamaoka, T., Yoshimoto, K, and Itakura, M. (1996) Molecular cloning of a human cDNA encoding a trifunctional enzyme of carbamoyl-phosphate synthetase - aspartate transcarbamoylase - dihydroorotase in de novo pyrimidine synthesis. Biochemical and Biophysical Research Communications, 219:249-255.
**Overexpression of CAD or parts of CAD**
   Davidson, J.N., Jamison, R.S. (1995) Expressing enzymatic domains of hamster CAD in CAD-deficient Chinese Hamster Ovary Cells. In: Purine and Pyrimidine Metabolism in Man VIII (Sahota A, Taylor M, Eds.) Plenum Press, New York, 1995, pp591-595.
Qui, Y and Davidson, J.N., (1998) CAD overexpression in mammalian cells. In: Purine and Pyrimidine Metabolism in Man IX (Greismacher A, Chiba P, Mueller M, eds.) Plenum Press, New York, 1998, pp 481-4.85.
Guy H.I., Bouvier A, and Evans D.R., (1997) The smallest carbamoyl-phosphate synthetase. J.B.C., 272:29255-29262
**PALA as inhibitor of CAD**
   **PALA can amplify CAD activity**
   Kempke, T.D., Swyryd, E.A., Bruist, M. and Stark, G.R. (1976) Stable mutants of mammalian cells that overproduce the first three enzymes of pyrimidine nucleotide biosynthesis. Cell, 9, 541-550
Chernove, O.B., Chernov, M.V., Ishizaka, Y., Agarwal, M.L. and Stark, G.R. (1998) MYC abrogates p-53-mediated cell cycle arrest in N-(Phosphonacetyl)-L-Aspartate-treated cells, permitting CAD gene amplification. Molecular and Cellular Biology, 18, 536-545
**CAD activity/regulation**
   Shaw, S.M and Carrey, E.A. (1992) Regulation of the mammalian carbamoylphosphate synthetase II by effecrors and phosphorylation. Eur. J. Biochem., 207, 957-965
Carrey, E.A. (1993) Phosphorylation, allosteric effectors and inter-domain contact in CAD; their role in regulation of early steps of pyrimidine biosynthesis. Biochemical Society Transactions, 21, 191-195.
Carrey, E.A. (1995) Key enzymes in the biosynthesis of purines and pyrimidines; their regulation by allosteric effectors and by phosphorylation. Biochemical Society Transactions, 23, 899-902.
   Irvine, H.S., Shaw, S.M., Patron, A. and Carrey, E.A. (1997) A reciprocal allosteric mechanism for efficient transfer of labile intermediates between active sites in CAD, the mammalian pyrimidine-biosynthetic multienzyme polypeptide. Eur. J. Biochem., 247, 1063-1073
**Background literature**
   Miltenberger, R.J., Sukow, K.A. and Farnham, P.J. (1995) An e-box-mediated increase in cad transcription at the G₁/S-phase boundry is suppressed by inhibitory c-Myc mutants. Molecular and Cellular Biology, 15, 2527-2535
Rao, G.N., Church R.L. and Davidson, J.N. (1988) Posttranscrptional regulation of the expression of CAD gene during differentiation of F9 teratocinoma cells by induction with retinoic acid and dibutyryl cyclic AMP. FEBS Letters, 232, 238-242
Rao, G.N. and Church, R.L. (1988) Regulation of CAD gene expression in mouse fibroblasts during the transition from the resting to the growing state. Experimental Cell Research, 178, 449-456
Bein K. and Evans, D.R. (1995). Stimmulation by 6-azauridine of *de novo* pyrimidine biosynthesis in BHK 165-23 cells. In: Purine and Pyrimidine Metabolism in Mann VIII (Sahota, A. Taylor, M., eds.) Plenum Press, New York, 1995, pp. 555-558
Bein K. and Evans, D.R. (1995). *De novo* pyrimidine nucleotide biosynthesis in synchronized Chinese Hamster Ovary cells. In: Purine and Pyrimidine
   Metabolism in Mann VIII (Sahota, A. Taylor, M., eds.) Plenum Press, New York, 1995, pp. 445-548
Evans, D.R., Bein, K., Guy, H.I., Liu, X., Molina, J.A. and Zimmermann, B.H. (1993) CAD gene sequence and the domain structure of the mammalian multifunctional protein CAD. Biochemical Society Transactions, 21, 186-191.
Guy, H.I. and Evans, D.R. (1998) Subdomain structure of carbamyl phosphate synthetase In: Purine and Pyrimidine Metabolism in Mann VIII (Griesmacher, A., Chiba, P., Mueller, M., eds.) Plenum Press, New York, 1998, pp. 265-269
Huisman, W.H., Raivio, K.O. and Becker, M.A. (1979) Simultaneous estimations of rates of pyrimidine and purine nucleotide synthesis *de novo* in cultured human cells. J. Biol. Chem., 254, 12595-12602
Carrey E.A. (1986) Nucleotide ligands protect the inter-domain regions of the multifunctional polypeptide CAD against limited proteolysis, and also stabilize the thermolabile pare-reactions of the carbamoyl-phosphate synthase II domains within the CAD polypeptide. Biochem. J., 236: 327-335
Coleman, P.F., Suttle, D.P. and Stark, G.R., (1978) Purification of a multifunctional protein bearing carbamyl-phosphate synthase, aspartate transcarbamylase, and dihydroorotase enzyme activities from mutant hamster cells. Methods in Enzymology, 51:121-134.
Mori, M. and Tatibana, M, (1978) A multienzyme complex of carbamoylphosphate synthase (glutamine): aspartate carbamoyltranferase: dihydroorotase (rat ascites hepatoma cells and rat liver). Methods in Enzymology, 51:111-121.
Kaseman, D.S., Meister A (1985) Carbamyl phosphate synthetase (glutamine-utilizing) from *E. Coli.* Methods in Enzymology, 113:305-326.
Guy, H.I., and Evans, D.R., (1997) Trapping an activated conformation of mammalian carbamoyl-phosphate synthetase. JBC, 272:19906-19912.
Guy H.I., Schmidt B, Herve G, and Evans, D.R., (1998) Pressure-Induced dissociation of carbamoyl-phosphate synthetase domains. JBC, 273:14172-14178.
Guy H.I., Rotger, A., and Evans, D.R., (1997) Activation by fusion of the glutaminase and synthetase subunits of E. Coli carbamyl-phosphate synthetase. JBC, 272:19913-19918.
Post, L.E., Post, D.J., and Raushel, F.M. (1990) Dissection of the functional domains of E. coli carbamoyl phosphate synthetase by site-directed mutagenesis. JBC, 265:7742-7747.

## Claims

1. A process for producing glycoprotein with enhanced glycosylation in a cell, the process comprising:
expressing glycoprotein in cells having enhanced CAD (carbamoyl phosphate synthetase II(CPSII)/aspartate transcarbamoylase (ATCase)/dihydroorotase multienzyme polypeptide complex) activity as compared to control host cells; and
thereby producing glycoproteins having enhanced glycosylation as compared to control host cells.

2. A process for producing glycoprotein with enhanced glycosylation in a cell, the process comprising the steps of:
selecting cells that are resistant to a CAD inhibitor and expressing glycoprotein in said resistant cells, wherein said expressing produces glycoprotein having enhanced glycosylation as compared to control host cells.

3. The process of claim 2, wherein the CAD inhibitor is N-phosphonacetyl-L-aspartate.

4. The process of claim 2, wherein the resistant cells are CHO cells or insect cells.

5. The process of claim 2, wherein the said expressing produces glycoproteins having enhanced galactosylation and/or sialylation as compared to control host cells.

6. The process of claim 1, wherein enhanced CAD activity is provided by the over expression of at least a portion of CAD.

7. The process of claim 1, wherein enhanced CAD activity is provided by the expression of a mutated CAD, wherein the mutation reduces inhibitory regulation by CAD's products.

8. The process of claim 7, wherein the mutated CAD reduces inhibitory regulation by UTP or UTP analogs.

9. The process of any one of the preceding claims, further comprising recovering the glycoprotein from the culture medium.

## Patentansprüche

1. Verfahren zur Herstellung von Glykoprotein mit erhöhter Glykosylierung in einer Zelle, umfassend:
- das Exprimieren von Glykoprotein in Zellen mit verstärkter CAD- (Carbamoylphosphatasesynthetase-II (CPSII)/Aspartattranscarbamoylase (ATCase)/Dihydroorotase-Multienzympolypeptidkomplex-) Aktivität im Vergleich zu Kontroll-Wirtszellen; und
- **dadurch** die Herstellung von Glykoproteinen mit erhöhter Glykosylierung im Vergleich zu Kontroll-Wirtszellen.

2. Verfahren zur Herstellung von Glykoprotein mit erhöhter Glykosylierung in einer Zelle, die folgenden Schritte umfassend:
- das Selektieren von Zellen, die gegenüber einem CAD-Inhibitor resistent sind, und das Exprimieren von Glykoprotein in diesen resistenten Zellen, worin diese Expression Glykoprotein bildet, das erhöhte Glykosylierung im Vergleich zu Kontroll-Wirtszellen aufweist.

3. Verfahren nach Anspruch 2, worin der CAD-Inhibitor N-Phosphonacetyl-L-aspartat ist.

4. Verfahren nach Anspruch 2, worin die resistenten Zellen CHO-Zellen oder Insektenzellen sind.

5. Verfahren nach Anspruch 2, worin die Expression Glykoproteine bildet, die erhöhte Glykosylierung und/oder Sialylierung im Vergleich zu Kontroll-Wirtszellen aufweisen.

6. Verfahren nach Anspruch 1, worin erhöhte CAD-Aktivität durch die Überexpression von zumindest einem Teil von CAD bereitgestellt wird.

7. Verfahren nach Anspruch 1, worin erhöhte CAD-Aktivität durch die Expression eines mutierten CAD bereitgestellt wird, worin die Mutation Inhibitionsregulation durch CAD-Produkte reduziert.

8. Verfahren nach Anspruch 7, worin der mutierte CAD Inhibitionsregulation durch UTP oder UTP-Analoga reduziert.

9. Verfahren nach einem der vorangehenden Ansprüche, weiters umfassend die Gewinnung des Glykoproteins aus dem Kulturmedium.

## Revendications

1. Procédé pour la production d'une glycoprotéine ayant une glycosylation améliorée dans une cellule, le procédé comprenant :
l'expression d'une glycoprotéine dans des cellules ayant une activité CAD (complexe polypeptidique multienzymatique carbamoyl-phosphate-synthétase II (CPSII)/aspartate transcarbamoylase (ATCase)/dihydro-orotase) améliorée comparativement aux cellules hôtes de contrôle ; et
la production, ainsi, de glycoprotéines ayant une glycosylation améliorée comparativement aux cellules hôtes de contrôle.

2. Procédé pour la production d'une glycoprotéine ayant une glycosylation améliorée dans une cellule, le procédé comprenant les étapes de :
sélection des cellules qui sont résistantes à un inhibiteur du CAD et expression de la glycoprotéine dans lesdites cellules résistantes, dans lequel ladite expression produit une glycoprotéine ayant une glycosylation améliorée comparativement aux cellules hôtes de contrôle.

3. Procédé selon la revendication 2, dans lequel l'inhibiteur du CAD est le N-phosphonacétyl-L-aspartate.

4. Procédé selon la revendication 2, dans lequel les cellules résistantes sont des cellules CHO ou des cellules d'insecte.

5. Procédé selon la revendication 2, dans lequel ladite expression produit des glycoprotéines ayant une galactosylation et/ou une sialylation améliorée(s) comparativement aux cellules hôtes de contrôle.

6. Procédé selon la revendication 1, dans lequel l'activité CAD améliorée est effectuée par la sur-expression d'au moins une partie du CAD.

7. Procédé selon la revendication 1, dans lequel l'activité CAD améliorée est effectuée par l'expression d'un CAD muté, dans lequel la mutation diminue la régulation inhibitrice des produits du CAD.

8. Procédé selon la revendication 7, dans lequel le CAD muté diminue la régulation inhibitrice par l'UTP ou des analogues de l'UTP.

9. Procédé selon l'une quelconque des revendications précédentes, comprenant en outre la récupération de la glycoprotéine à partir du milieu de culture.
